(19) **Europäisches Patentamt / European Patent Office / Office européen des brevets**

(11) **EP 3 130 566 A1**

(12) **EUROPEAN PATENT APPLICATION**
published in accordance with Art. 153(4) EPC

(43) Date of publication:
**15.02.2017 Bulletin 2017/07**

(21) Application number: **14888970.2**

(22) Date of filing: **15.04.2014**

(51) Int Cl.:
$C02F\ 3/00$ (2006.01)    $G21F\ 9/04$ (2006.01)
$C12N\ 5/00$ (2006.01)

(86) International application number:
**PCT/RU2014/000273**

(87) International publication number:
**WO 2015/156698 (15.10.2015 Gazette 2015/41)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **11.04.2014 ES 201430540**

(71) Applicant: **Kornilova, Albina Aleksandrovna Moscow 125040 (RU)**

(72) Inventor: **VYSOTSKII, Vladimir Ivanovich Kiev 03057 (UA)**

(74) Representative: **Spengler, Robert Potthast & Spengler Patentanwälte Küfergasse 11 89073 Ulm (DE)**

(54) **METHOD FOR PURIFYING WATER OF RADIONUCLIDES**

(57) The invention relates to the field of water purification. The present method for purifying water of radionuclides by nuclear transmutation involves preparing a culture medium for the growth of microbiological cultures, adding a biomass of microorganisms to said medium, holding the biomass of microorganisms in an aqueous solution undergoing purification for from 10 hours for aerobic microorganisms to 24 hours for anaerobic microorganisms, and adding key trace elements and/or combinations thereof to different parts of the resultant aqueous solution. The aqueous solution undergoing purification is held for the purpose of selecting the trace elements and/or combinations thereof necessary to speed up the process of transmutation, and the selected trace elements and/or combinations thereof are added to the aqueous solution undergoing purification. The aqueous solution undergoing purification is held for the time necessary to achieve the required residual radioactivity value, and the biomass of microorganisms is removed from the aqueous solution undergoing purification.

**EP 3 130 566 A1**

**Description**

The present invention relates to the field of liquid radioactive waste treatment, and more particularly to methods for purifying water from radionuclides.

The purification of radionuclide-contaminated water is a highly serious challenge for environmental protection. Such water results both from scheduled process operations due to the specificity of nuclear-oriented production activities and from contingencies that are particularly due to emergencies nuclear-oriented facilities may cause.

Nuclear fission reaction in nuclear reactors produces a large number of medium-lived or long-lived radioactive isotopes and isomers (high radioactive waste) as follows: H.sup.3, Be.sup.7, Csup.14, F.sup.18, Na.sup.22,24, Si.sup.31, P.sup.32,33, S.sup.35, Cl.sup.36,38, K.sup.42,43, Ca.sup.45,47, Sc.sup.46,47,48, V.sup.48, Cr.sup.51, Mn.sup.51,52, 52m,53,54,56, Fe.sup.52,55,59, Co.sup.55,56,57,58,58m,60,60m,61,62m, Ni.sup.59,63,65, Cu.sup.64, Zn.sup.65,69, 69m, Ga.sup.672, Ge.sup.671, As.sup.73,74,76,77m, Se.sup.75, Br.sup.82, Rb.sup.86, Sr.sup.85,85m,87m,89,90,91, 92, Y.sup.90,91,91m,92,93, Zr.sup.93,95,97, Nb.sup.93m,94,95,97,98, Mo.sup.90,93,99,101, Tc.sup.96,96m,97,97m, 99,99m, Rh.sup.97,103,105,106Ru,103m,105, Pd.sup.103,109, Ag.sup.105,110m,111, Cd.sup.109,115,115m, In.sup.111,113m,114m,115m, Sn.sup.113,125, Sb.sup.122,124,125, Te.sup.123m,125m,127,127m,129,129m,131, 131m,132,133,133m,134, I.sup.123,125,126,129,130,131,132,133,134,135, Cs.sup.129,131,132,134,134m,135,136, 137,138, Ba.sup.131,140, La.sup.140, Ce.sup.139,141,143,144, Pr.sup.142,143, Nd.sup.147,149, Pm.sup.147,149, Eu.sup.151,153Sm, 152,152m,154,155, Gd.sup.153,159, Tb.sup.160, Dy.sup.165,166, Ho.sup.166, Er.sup.169,171, Tm.sup.170,171, Yb.sup.175, Lu.sup.177, Hf.sup.181, Ta.sup.182, W.sup.181,185,187, Re.sup.186,188, Os.sup.185, 191,191m,193, Ir.sup.190,192,194, Pt.sup.191,193m,197,197m, Au.sup.198,199, Hg.sup.198,199, Pb.sup.200,201, 202,204T1,203, Bi.sup.206,207, Po.sup.203,205,207, At.sup.211, Ra.sup.225,227, Th.sup.226,229, Pa.sup.230,233, U.sup.230,231232,233,236,237,239,240, Np.sup.237,239,240, Pu.sup.234,235,236,237,238,239,240,241,242,243, 244, Am.sup.241,242,242m,243, Cm.sup.242,243,244,245,246,247,248, Bk.sup.249, Cf.sup.246,248,249,250,251, 252,253,254, Es.sup.253,254,254m, Fm.sup.254,255.

Sr.sup.90, Tc.sup.99,99m, Cs.sup.137, Np.sup.237,239, Pu.sup.238,239,240,241, Am.sup.241, and Cm.sup.242 isotopes whereof impose the highest biohazard risks.

There has been proposed a method of biological removal of technogenic radionuclides from water (Patent RU2255906 published on July 10, 2005), which is based on high absorption properties of algae (namely, *Phillophonia elongata, Ulwa regida, Thalassiosira,* etc.), transplant seedlings of which are placed into biocontainers that are mounted into a reservoir of water to be decontaminated. A metabolism process developed when sprouting said algae for 20-40 days induces an active absorption based on metabolic and biochemical processes, and the accumulation of some radionuclide types, which leads to water purification. When filled with growing algae the biocontainer is retrieved, and the algae are dried and burned with radioactive ash residues being buried. The maximum radionuclide build-up factor for said algae (ratioed to identical volume of water) is 30 (*for Cs.sup.137*) and 40 (*for Sr.sup.90*).

It has been found that this method does not provide for the deactivation of radionuclides as hazardous radionuclides are transformed (reduced to ash) instead of being destroyed.

It has formerly been proposed to dispose of radioactive waste by exposing it to irradiation by thermal neutron flux (Patent US4721596 A published January 26, 1988). Said irradiation induces the capture of neutrons by nuclei of long-lived isotopes and a further chain of nuclear transmutations generating other isotopes that can in particular be more short-lived, which thus shortens the duration of spontaneous decay-induced natural deactivation.

The drawback of the above method is that fission reactions when influenced by slow neutrons can only proceed in certain heavy radioactive isotopes (U.sup.235 and Pu.sup.239 in particular), and, besides, the absorption of such neutrons by stable nuclei of mass-average elements may generate radioactive isotopes of these nuclei.

The object of the present invention is to provide a method for purifying water solutions containing radionuclides that involves a transmutation phenomenon (nuclear transformation isotope transmutation) consisting in that ions of radioactive isotopes (radionuclides) transmute to other stable isotopes in growing microbiological cultures.

Said object is achieved by creating conditions in a water solution to be purified to allow nuclear transmutation whereby radioactive isotopes of one chemical elements transmute to non-radioactive isotopes of other chemical elements in growing microbiological cultures, which requires that a nutrient medium is prepared to allow growth of microbiological cultures and that said nutrient medium is deficit in the chemical element corresponding to the isotope resulting from said transmutation (Isotope 1) and that said nutrient medium contains all primary isotope components (Isotope 2) required for said transmutation, wherefore microbiological cultures requiring Isotopes 1 for their growth and development are grown in said nutrient medium, wherefore a biomass of microorganisms including viable microbial syntrophic associations is added therein, for example, in the form of granules, whereupon said nuclear transmutation proceeds in three stages. wherein the first stage comprises stimulating the effect of mutagenic adaptation of microorganisms contained in the biomass to given radionuclide types (Isotopes 3) present in said water solution to be purified, which is carried out by keeping the microorganism biomass in a stepwise manner in a liquid containing water in the amount sufficient for covering the volume of the biomass and that of said nutrient medium, at a temperature (increased to 30-40°C) that best accelerates mutagenesis, within a time interval of 10 hours for aerobic organisms and 24 hours for anaerobic organisms, with the amount of said liquid being progressively increased by adding portions of water solution to be purified containing radionuclides that do not provoke any demise of the biomass due to radioactive irradiation, and by having said water solution achieve target concentration, whereupon the biological part of the transmutation process is optimized during the second stage by adding key necessary microelements and/or combinations of these microelements in a separate manner in small different parts of the solution obtained, and by keeping equal quantities of the biomass that underwent mutagenic adaptation in those different parts throughout a certain period of time, and by selecting those microelements and/or their combinations that accelerate transmutation to the maximum extent, whereupon microelements selected and/or their combinations selected are added in the purifiable water solution during the third stage in the amount that ensures the maximum transmutation rate for radioactive isotopes within the entire volume of the water solution to be purified, whereupon the biomass is extracted therefrom. Duration of the third stage is a function of time required for the water solution to achieve target value of residual radioactivity. The present invention prevents the microorganism biomass from absorbing radionuclides in a direct way without transmutation of the latter, which is achieved by adding sufficient quantities of those microelements that are needed for the growth of the biomass and represent stable analogues (stable isotopes) of those radionuclides that are to be disposed of to said water solution to be purified or to said biomass.

One embodiment provides for the granulation of said microorganism biomass carried out after the latter has bonded with said nutrient medium, in the presence of compounds forming stable non-soakable granules, which structures allow for a free movement of water and salts and radionuclides dissolved therein within the entire volume of granules.

Another embodiment provides for the formation of said microorganism biomass using syntrophic associations of aerobic and anaerobic microorganisms.

To purify solutions containing seawater (not fresh water) or based thereon from radionuclides, it is preferred to use the biomass of microorganisms that includes viable microbial syntrophic associations adapted to seawater, and, for instance, based on viable silts for which seawater is a natural habitat.

It is preferred that the third transmutation stage comprises carrying out a continuous mixing of said water solution and said biomass and/or bubbling through said water solution and said biomass.

It is preferred that when purifying water solutions from radionuclides $Cs^{137}$ and $Sr^{90}$ by transmuting the latter into stable isotopes of other chemical elements, elements $Mg$ and $K$ are removed from the nutrient medium (or their concentration therein is lowered).

The above novel features allow performing the method of purifying water solutions containing radionuclides of one chemical elements by transmuting the former into stable isotopes of other chemical elements.

Fig. 1 shows a gamma-ray spectrum of reactor isotopes the purifiable water contains on the 10th day after its extraction from the core of a pressurized water reactor.

Fig. 2 shows a plotted dependence of activity Q(t) of $La^{140}$ reactor isotope present in reactor water samples during the transmutation experiment ($Q_{cultures}$ activity measured in cuvettes in the presence of granules containing syntrophic associations of metabolically active microorganisms) and in control cuvettes ($Q_{control}$ activity) free of any microorganisms.

Fig. 3 illustrates an accelerated disposal (deactivation) of $Cs^{137}$ isotope in "biological cells" in the presence of microbiological granules and various chemical elements. 1- $Cs^{137}$ (control), $\tau^* \approx 30$ years; 2 - $Cs^{137}$ + granules+KCl, $\tau^* \approx 10$ years; 3 - $Cs^{137}$+granules+ NaCl, $\tau^* \approx 480$ days; 4 - $Cs^{137}$+ granules, $\tau^* \approx 380$ days; 5 - $Cs^{137}$+ granules + $CaCO_3$, $\tau^* \approx 310$ days.

Fig. 4 depicts a principle of formation of giant fluctuations of kinetic momentum and kinetic energy in a coherent correlated state (right).

Two factors influence the transmutation of isotopes in growing microbiological culture and syntrophic association of such cultures.

The first factor relates to biological processes involving the use and fixation of chemical elements during metabolic processes, and the second one pertains to physical processes of nuclear transformation stimulated by biological processes.

A growth of any biological object requires a strongly defined set of chemical microelements and macroelements. A mere lack of one of said elements will slow the growth.

Such vital elements are
O (a typical concentration in a living culture is 24%), H (about 64%), C (about 9%), and N (about 0.13%)).

The microelements required for the growth of various biological cultures are as follows:

Na ($7.10^{-3}$ %), K ($4,5.10^{-2}$ %), Ca ($7,5.10^{-2}$ %), Mg ($2.10^{-2}$ %), Fe ($8.10^{-4}$ %),
P ($1,3. 10^{-2}$ %), Si ($3,5.10^{-2}$ %), Cl ($7.10^{-3}$ %), Al ($6.10^{-3}$ %), B ($6.10^{-4}$ %), Ti ($10^{-4}$ %),
Zn ($3.10^{-5}$ %), Li ($10^{-4}$ %), Cu $10^{-5}$ %), Sr ($10^{-5}$ %), Ba ($5.10^{-6}$ %), F ($3.10^{-5}$ %),

Br ($6.10^{-6}$%), Rb ($4.10^{-6}$%), Sn ($10^{-6}$ %), Ni ($5.10^{-6}$ %), Mo$10^{-6}$ %), Co$10^{-6}$ %).

The above concentrations are typical although they may be several times as different for various cultures.

Also, S, Mn, J, Hg, and other microelements are needed by some microbiological cultures.

Where any of the chemical elements needed are missing, they can be substituted by their biochemical (stereochemical) analogues that have similar ionic radii and identical (or similar) valence values. Where, in particular, atoms of a chemical element needed are missing in the nutrient medium, though can be formed during the nuclear fusion of relevant nuclides, a newly formed nucleus of such chemical element needed with its electron environment is immediately incorporated after the fusion into the growing culture. The same occurs when in the no-atom condition the fusion results in the formation of a stereochemical analogue of a chemical element needed.

Said "incorporation" of the synthesized element (isotope 1) into the growing microbiological system is actually a fixation process and confirms the irreversibility of said fusion. Nuclear fusion proceeds in the presence of initial nuclei through, for example, a short-term fluctuation of energy $\delta E$ over time $\delta t$. If said fluctuation is enough to overcome a Coulomb barrier and the energy released in the reaction during time $\delta t$ is $\Delta E > \delta E$, the fusion reaction becomes irreversible. Otherwise, if $\Delta E < \delta E$, the reaction is reversible and does not lead to the formation of the isotope required.

A further object specific to the advantages of the method disclosed herein is to use, not pure microbiological cultures but syntrophic microbiological associations that include many thousands of various types of microorganisms pertaining to various physiological groups, which represent various groups of microbial metabolism and feature different mechanisms of microbial accumulation.

These microorganisms are not in the form of a simple mechanical mixture. They coexist in the syntrophic association in a state of symbiosis where they actually form a single microorganism though the latter features separate internal metabolism systems. Each member and each physiological group of such community is best adapted to coexistence and is in a state of collective mutual assistance and mutual defence. This system is highly adaptable to various variances and "aggressive" impacts of external environment (particularly, to a high radiation exposure level, presence of toxins, or to low $pH$ values).

Each type of a particular biochemical environment provides the most favourable conditions for microorganisms of a particular physiological group. All other groups of the syntrophic association thus play an auxiliary role and work for the leader that progresses to the maximum efficiency. With changes in external conditions (temperature change, compositional change of nutrient medium, action of toxins and ionising radiation, additional effect of free radicals, etc.), such leader role may well pass on to another physiological group that is best adapted to changed conditions. Ex-leaders now become members to collective aid and contribute to the development of the leading group and to that of the association as a whole.

Such system is found to be best adapted to changing aggressive conditions, which results in the microbial growth including that observed under radiation conditions. The efficiency of such "collective defence" is extremely high. For instance, it is known that no "pure" microorganism strains can develop in acidic medium where $pH =2$ (concentrated hydrochloric acid). Syntrophic association, however, successfully grows and spreads in said medium, following a certain transitional period of adaptation. The time interval of the full adaptation covers the alternation of 5 to 10 generations and therefore is considered to last from 10 hours to 10 days.

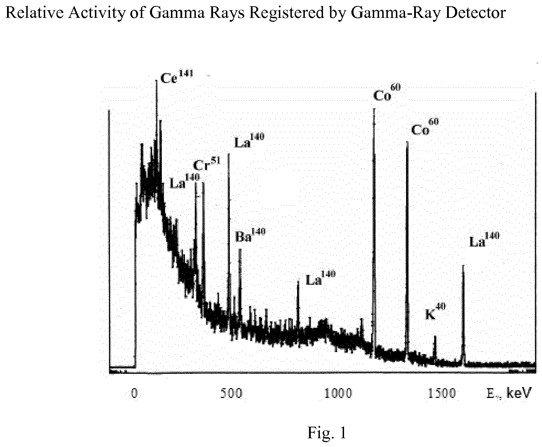

The experimentally studied water had an activity of about $10^{-4}$ Ci/l and contained a number of high-activity unstable isotopes (namely, Na.sup.24, K.sup.40, Co.sup.60, Sr.sup.90, 91., I.sup.131, Xe.sup.135, Ba.sup.140, La.sup.140, Ce.sup.141, Np.sup.239). See Fig. 1.

Water samples equal in volume (about 5 ml) were placed into similar capped thin-wall glass cuvettes of about 10 ml each. Amounts of granulated microorganism biomass equal in mass were placed into a part of cuvettes containing radioactive water. The remaining cuvettes that contained radioactive water without the granulated biomass were classified as control ones.

The research involved the analysis of the change in the amplitude of spectral lines which energy exceeds 500 keV. This was made to raise accuracy as the Compton background and the absorption of water contained in a cuvette have a significant influence on low-energy rays.

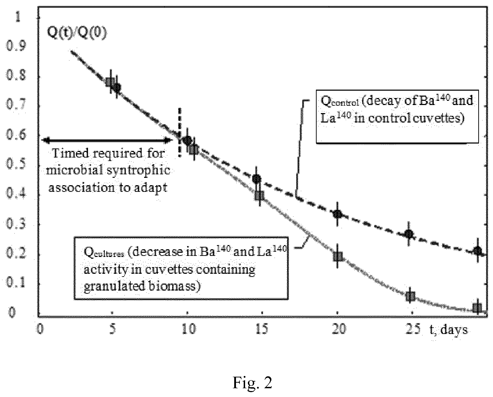

Fig. 2 depicts averaged behaviour of $La.sup.140$ isotope activity in experimental cuvettes ($Q_{cultures}$) and control cuvettes ($Q_{control}$) versus time counted after the start of the experiments. This isotope has a relatively short lifetime ($\tau_{La}$ = 40.3 h), results from beta decay $Ba^{140} \rightarrow L_a^{140} + \beta^- + \tilde{v}$ and is a daughter isotope of a more long-lived isotope $Ba.sup.140$ which lifetime is $\tau_{Ba}$ = 12.7 days.

Initial specific activity values for isotopes $Ba.sup.140$ and $La.sup.140$ (on the 10th day after the water was extracted from the core of the reactor) were $Q_{Ba140} / V$ = 5400 $bk/l$ and $Q_{La140} / V$ = 8500 $bk/l$, respectively, for each cuvette. Since ($\tau_{La} \ll \tau_{Ba}$), the observed decrease in $La.sup.140$ activity revealed that in $Ba.sup.140$ activity.

The decrease in $La.sup.140$ activity in control cuvettes was found to roughly follow the law of "conventional" decay for $Ba.sup.140$ isotope with the "tabulated" lifetime value. The same law of decrease in $La.sup.140$ activity was observed in granule-containing cuvettes until the 10th day of the experiment. Following this initial period of adaptation, consecutive measurements showed that decrease rate for *La.sup.140* activity (and consequently, *Ba.sup.140* activity) represented an accelerated decay (was equivalent thereto by the law of activity change). Extrapolation showed the effective lifetime of this isotope was reduced by about a factor of two compared to the lifetime of *Ba.sup.140.*

These results can be explained by assuming that the radioactive isotope *Ba.sup.140* contained in the experimental cuvette transmutes to non-radioactive isotope of another chemical element. The fact that the decay law reveals an initial constant area can be explained by the above-mentioned processes in which the microbiological association present in the cuvette containing active water adapts to the radioactive irradiation. The time for said adaptation (about 10 days) well correlates with that expected for the alternation of 5 to 10 generations of microbial cultures.

The analysis of possible isotope transmutations showed in this case the radioactive isotope *Ba.sup.140* may transmute to a stable nucleus of another type in the following way

$$Ba^{140} + C^{12} \rightarrow Sm^{152}$$

This transmutation reaction is energy useful and characterized by positive energy. Carbon required for this reaction to occur exists in abundance in the microbiological granules.

Following the law of constant composition of biological substances which stands to be one of the fundamental properties of the living matter, the isotope transmutation reaction occurring in the biological system is only possible if this reaction produces an isotope corresponding to a chemical element that either ranks itself among necessary chemical elements or is a biochemical analogue of such element. In the latter case, it should have nearly identical ionic radius and preferably identical valence. Moreover, if the nutrient medium contains no or low amounts of a necessary chemical element, said reaction will be highly efficient.

Comparison of ions $Sm^{2+}$ and $Ca^{2+}$ shows that they are biochemical analogues and have nearly identical ionic radii in a bivalent state ($R_{Sm} \approx 1.2$ A, $R_{Ca} \approx 1.06$ A). Calcium ranks among the necessary chemical elements, and if its concentration was low within the entire volume of the microbiological granules, it can be argued that the microbiological association could compensate for the lack of calcium by synthesizing its biochemical analogue (samarium).

It should be noted that the granulated biomass which formation is based on natural syntrophic associations that are generated in fermented animal feces or in natural silts, may have different chemical composition. To optimize the growth of this biomass, a balanced complex of key macroelements and microelements is required. The composition of the latter can only be defined experimentally by independently adding key microelements and analyzing transmutation efficiency changes resulting therefrom.

The research involved the use of identical glass cuvettes, each containing 10 ml of distilled water which comprised *Cs.sup.137* solution with a specific activity of

$$Q_{Cs^{137}} \approx 2.10^6 \, bq/l$$

7 cuvettes contained granules in equal amounts. Refined salts of K, Ca, Na, Fe, Mg and P were added to 6 active water-containing cuvettes respectively. These chemical elements rank among the ones required for any biological system to grow. Two additional cuvettes were used for control purposes: one contained radioactive water and granules (and no additional salts), another had radioactive water only.

All cuvettes were capped and stored at a temperature of 20°C. The same detector was used to measure the amplitude spectrum of gamma rays every 7 days. Particular attention was paid for reducing the influence of measurements uncertainties. To do that, low-height cuvettes and a detector with a Ge crystal of larger diameter were used. Each measurement was made by placing cuvettes in the same way in the centre of the detector crystal.

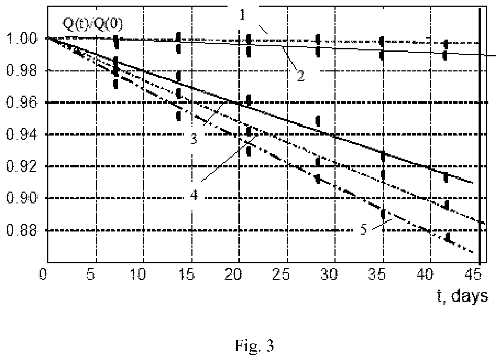

Measurement results for *Cs. sup.137* isotope activity are shown in Fig. 3.

For the control cuvette containing radioactive water only, the change in *Cs.sup.137* isotope activity represented a conventional spontaneous decay with a lifetime of about 30 years.

The cuvette containing calcium salt only showed the most rapid decrease in activity (it was equivalent to a 35-fold reduction of lifetime down to $\tau^* \approx 310$ days). The cuvette containing additional calcium salt showed a decrease in *Cs.sup.137* activity that corresponded to a 10-year lifetime. Such decrease was not due to the accelerated decay but was induced by the disposal reaction wherein *Cs.sup.137* transmuted to a stable isotope of another element.

The suggested disposal of *Cs^137* radionuclide is due to the reaction

$$Cs^{137} + p \rightarrow Ba^{138},$$

involving the participation of water protons. Said reaction produces a stable isotope *Ba.sup.138.*

Ions $Ba^{2+}$ and $K^+$ are biochemical analogues and have nearly identical ionic radii in a bivalent state ($R_{Ba} \approx 1.4$ A, $R_K \approx 1.33$ A). Since the substituted element (potassium) is among the necessary microelements, its substitution is highly possible, and ions of synthesized barium can replace those of potassium in metabolic processes during the growth of cultures. This substitution appears more efficient than a "direct" substitution of potassium with cesium (this is well seen from a large difference between the radius of $R_{Cs} \approx 1.65$-$1.69$ A and $R_K \approx 1.33$ A). It should be noted that such ion substitution was earlier observed and analysed in experiments with *Blastocladiella emersonii* microbial culture [Van Brunt J., Caldwell J. H., Harold F. M. Circulation of potassium across the plasma membrane of Blastocladiella emersonii: K-chanel // J. Bacteriol., 1982, v.150, N 3, pp. 1449-1561]. These experiments registered the substitution of $K^+$ ions with $Rb^+$ and $Ba^{2+}$ ions. These ions can replace each other in processes relating to the transfer of ions across the membrane to a cell.

Another very hazardous radionuclide produced during the fission and contained in spent nuclear fuel is isotope *Sr.sup.90.* Said isotope can be disposed of by transmuting it to various stable isotopes of other elements in one of the reactions.

$$_{38}Sr^{90} +_{6} C^{12} =_{44} Ru^{102} ,$$

$$_{38}Sr^{90} +_{8} O^{16} =_{46} Pd^{106} ,$$

$$_{38}Sr^{90} +_{11} Na^{23} =_{49} In^{113}$$

The latter produce stable isotopes *Ru, Pd* and *In* that are biochemical analogues of the necessary chemical elements such as *Fe* and *Mg , Ca* and *Mg , Fe* and *Mg* respectively. Such conformity is due to equal ionic radii of the above elements

$$R_{Ru}^{3+} = 0.77A; R_{Fe}^{3+} = 0.6-0.67; R_{Mg}^{2+} = 0.7-0.78A;$$

$$R_{Pd}^{2+} = 0.85-0.88\,A; R_{Ca}^{2+} = 0.96-1.04; R_{Mg}^{2+} = 0.7-0.78A;$$

$$R_{In}^{3+} = 0.8-0.9\,A; R_{Fe}^{2+} = 0.75-0.83; R_{Mg}^{2+} = 0.7-0.78A$$

When absent, said necessary chemical elements can be substituted by stable isotopes of *Ru, Pd* and *In* elements. It is apparent that all these elements are biochemical analogues of *Mg* and *Fe*. Hence, if purifiable water and active medium contain no *Mg* and *Fe*, the latter may be replaced with products of all three possible reactions providing the disposal of radioactive strontium.

The reason for which the efficiency of the disposal reaction rises with the use of additional calcium salt lies in the general law of metabolism of microbiological cultures: the optimal growth of a culture requires a balance of all macroelements and microelements. The calcium deficit was presumably the bottleneck that slowed the growth and the associated transmutation in a particular growing microbiological system. It is evident that when using granules prepared using other natural syntrophic associations the effect of different salts may be different and has to be experimentally determined.

To allow two nuclei to interact, it is necessary to provide conditions to overcome the Coulomb barrier that prevents the nuclei from coming close to each other. The barrier is very high, and its transparency (for the model case with no atomic electrons) can be defined by the dependence

$$D \approx \exp(-2\pi Z_1 Z_2 / \hbar v) ,$$

derived by the Gamow formula

$$D = \exp(-2\hbar \int \sqrt{2m\{V(r)-E\}} \, dr / \hbar),$$

which depends on total $E = \mu v^2 / 2$ and potential (Coulomb) energies $V(r) = Z_1 Z_2 e^2 / r$ of mutual repulsion of nuclei and charges $Z_1 e$ and $Z_2 e$. Here, $\mu = m_1 m_2 / (m_1 + m_2)$ is a "reduced" mass of the interacting nuclei which is a function of the mass of each nucleus. When particles are in thermal equilibrium, $v \approx <v> = \sqrt{kT/4\mu}$.

The consideration of the shielding effect of atomic electrons reduces barrier width, and hence raises transparency factor allowing to let additional effective energy in, which represents the substitution $E = kT + E_{eff}$. When hydrogen atoms interact $E_{eff} \approx 27 \, eV$. In a biological system $E = E_{eff}$, and the barrier transparency has a very low value $D \approx 10^{-100}$.

This probability is much less when heavier nuclei interact. For instance, where cesium and hydrogen nuclei interact $E_{eff} \approx 300 eV$, the transparency of the barrier at $kT << E_{eff}$ is $D \approx 10^{-1000}$. Nearly the same negligible probability is relevant to the case where one or the two interacting particles (interacting nuclei with electron environment) are in a stationary potential well.

These estimations show that no "conventional" nuclear reactions are possible in living organisms at the temperature typical for the growth of microbiological cultures. Such conclusion correlates with the case where pairwise nuclear interaction in free space or in stationary potential well is considered.

The author's works (V.I.Vysotskii, M.V.Vysotskyy. "Coherent correlated states and low-energy nuclear reactions in non stationary systems". European Physical Journal. A, 2013, v.49, issue 8: 99; and V.I.Vysotskii, S.V.Adamenko, M.V.Vysotskyy. 2013. "Acceleration of low energy nuclear reactions by formation of correlated states of interacting particles in dynamical systems", Annals of Nuclear Energy, 2013, v.62, 618-625) show that a certain non-stationary deformation of potential well in which even one of the interacting particles is located leads to a significant increase in nuclear barrier transparency. Such effect is due to the formation of coherent correlated states of a particle featuring the synchronization effect and the efficient summation (interference) of fluctuations of different components of particle's momentum in a non-stationary superpositional state. Said summation generates large final fluctuations of total momentum and fluctuations of particle's kinetic energy, which contributes to a significant increase in potential barrier's transparency factor and hence to similar increase in the probability of nuclear fusion reaction. A simple interpretation of this quantum-mechanical phenomenon is as follows:

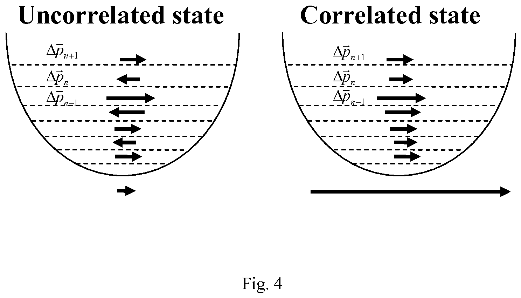

In the superposition state a particle may be at different energy levels $E_n$ of potential well with different probabilities (see Fig. 4).

In this system, dispersion of particle's total momentum and mean kinetic energy are defined by the formulas

$$\sigma_p \equiv < \{\vec{p}(t) - <\vec{p}(t)>\}^2 > = < \left\{ \sum_n^N \Delta\vec{p}_n(t) \right\}^2 > = N \left\langle (\Delta\vec{p}_n)^2 \right\rangle + N^2 \left\langle \Delta\vec{p}_n \Delta\vec{p}_m \right\rangle,$$

$$<T> = \sigma_p / 2m$$

When in a "conventional" state (i.e. non-coherent uncorrelated state), momentum fluctuations are mutually independent at different levels and $\langle \Delta\vec{p}_n \Delta\vec{p}_m \rangle = 0$. Here, particle's mean kinetic energy is defined by the quantity $< T_{noncorr} > = N \langle (\Delta\vec{p}_n)^2 \rangle / 2m = N < T_n >$.

This relation meets standard perspectives that in a system of quantum levels particle's mean kinetic energy equals the sum of mean energies at these levels in a potential well.

When in a coherent correlated state $\langle \Delta\vec{p}_n \Delta\vec{p}_m \rangle \neq 0$, mean kinetic energy is

$$<T_{corr}> = \sigma_p / 2mN = \left\langle (\Delta\vec{p}_n)^2 \right\rangle / 2m + N^2 \left\langle \Delta\vec{p}_n \Delta\vec{p}_m \right\rangle / 2m =$$
$$<T_{noncorr}> + N^2 \left\langle \Delta\vec{p}_n \Delta\vec{p}_m \right\rangle / 2m$$

In this case a significant rise in mean kinetic energy occurs in the multilevel system.

In particular, if

$$\left\langle (\Delta \vec{p}_n)^2 \right\rangle / 2m = \left\langle \Delta \vec{p}_n \Delta \vec{p}_m \right\rangle / 2m = <T> / N \;,$$

, $<T_{noncorr}> = N<T_n>$ will be relevant for non-coherent uncorrelated state and $<T_{corr}> = (N+1) <T_{noncorr}>$ will refer to coherent correlated state.

It is apparent that mean kinetic energy increases by $N+1 >> 1$ times. The presence or the absence of such giant momentum fluctuations is schematically shown by a large vector under the right figure in Fig. 4 and by a very small vector under the left figure. The relation between these fluctuations is several-times as large in real situation.

Formally, the existence of this state is characterized by a correlation factor defined by the formula

$$r(t) = <q\hat{p} + \hat{p}q> / 2\delta q \delta p, \quad \delta q = \sqrt{\sigma_q}, \quad \delta p = \sqrt{\sigma_p} \;,$$

as well as by an uncertainty relation (Schrödinger-Robertson uncertainty relation)

$$\delta q \delta p \geq \hbar / 2\sqrt{1 - r^2}$$

The quantity $|r|$ varies over the interval $0 \leq |r| \leq 1$. With no correlation between coordinate $q$ and particle's momentum $p$, we have $r = 0$, and the latter formula is thus reduced to Heisenberg uncertainty relation $\delta q \delta p \geq \hbar / 2$.

In the extreme case of a totally correlated state $|r| \to 1$, dispersion of particle's momentum becomes unrestrictedly large, and transparency factor $D$ of any potential barrier rises to the maximum of $D \to 1$ at an arbitrary low energy of the particle.

The works show that a monotone contraction of the potential well comprising one of the interacting particles makes the correlation factor rise to $|r| \geq 1 - 10^6$ letting the transparency factor rise many times as high as from the extreme low values $D_{noncorr} \approx 10^{-100}...10^{-1000}$ for "conventional" (uncorrelated) particle's states to a value of $D_{corr} \to 1$.

Local discontinuities of the growth and dynamic character of biophysical phenomena (cell division, DNA replication, etc.) inevitably give rise to said potential wells in the growth area of any biological object wherein said wells exist for a certain time and disappear due to the influence of random collisions of atoms and molecules.

Particles present therein can have themselves formed coherent correlated ones for a short time in every such potential well. If the both nuclei that are potentially suitable for the fusion required are present (for instance, strontium and hydrogen atoms), the probability of $Cs^{137} + p = Ba^{138}$ radionuclide disposal reaction is very strong. Fast-growing biological systems exhibit a continuous reproduction of a large number of such contracting potential wells each of which is a one-time nuclear microreactor if all the conditions required are available. This effect is impossible in static systems.

## Claims

1. A method of purifying water from radionuclides consisting in creating conditions in a water solution to be purified to allow nuclear transmutation whereby radioactive isotopes of one chemical elements (Isotope 3) transmute into non-radioactive isotopes of other chemical elements (Isotope 1) in growing microbiological cultures, which requires that a nutrient medium deficit in the chemical element corresponding to Isotope 1 and containing all primary isotope components (Isotope 2) required for said nuclear transmutation is prepared to allow growth of microbiological cultures, whereafter a biomass of microorganisms is added in said nutrient medium and said nuclear transmutation proceeds in three stages, wherein the first stage comprises stimulating the effect of mutagenic adaptation of microorganisms contained in the biomass to Isotopes 3 by keeping the microorganism biomass in a stepwise manner in a liquid containing water in the amount sufficient for covering the volume of the biomass and that of said nutrient medium, within a time interval of 10 hours for aerobic organisms and 24 hours for anaerobic organisms, with the amount of said liquid being progressively increased by adding portions of water solution to be purified containing radionuclides that do not provoke any demise of the biomass due to radioactive irradiation, and by having said water solution achieve target concentration, whereupon the biological part of the transmutation process is optimized during the second stage by adding key necessary microelements and/or combinations of these microelements in a separate manner in small different parts of the solution obtained, and by keeping equal quantities of the biomass that underwent mutagenic adaptation in said small different parts throughout a certain period of time, and by selecting those microelements and/or their combinations that accelerate transmutation to the maximum extent, whereafter during the third stage microelements selected and/or their combinations selected are added in the purifiable water solution in the amount that ensures the maximum transmutation rate for isotopes 3 within the entire volume of the water solution to be purified, and the exposure lasts until the water solution achieves target value of residual radioactivity, whereupon the biomass is extracted therefrom.

2. A method as recited in claim 1, *wherein* the first stage comprises keeping the microorganism biomass at a temperature of 30-40°C.

3. A method as recited in claim 1, *wherein,* after adding the microorganism biomass to the nutrient medium, the obtained matter is granulated in the presence of compounds forming stable non-soakable granules which structures allow for free movement of water and salts and radionuclides dissolved therein within the entire volume of granules.

4. A method as recited in claim 1, *wherein* the microorganism biomass is formed using syntrophic associations of aerobic and anaerobic microorganisms.

5. A method as recited in claim 1, *wherein* the purification of seawater-containing solutions from radionuclides involves the use of a biomass of microorganisms that includes viable microbial syntrophic associations adapted to sea water, and, for instance, based on viable silts for which sea water is the natural habitat.

6. A method as recited in claim 1, *wherein* the third transmutation stage comprises carrying out a continuous mixing and/or bubbling through the water solution and the microorganism biomass.

7. A method as recited in claim 1, *wherein,* when purifying water solutions from radionuclides *sub.55.Cs.sup.137* and *sub.38.Sr.sup.90,* elements *Mg* and *K* are removed from the nutrient medium or their concentration therein is lowered.

Relative Activity of Gamma Rays Registered by Gamma-Ray Detector

Fig. 1

Fig. 2

Fig. 3

Fig. 4

# EP 3 130 566 A1

## INTERNATIONAL SEARCH REPORT

| | International application No. |
| --- | --- |
| | PCT/RU 2014/000273 |

**A. CLASSIFICATION OF SUBJECT MATTER**

C02F 3/00 (2006.01); G21F 9/04 (2006.01); C12N 5/00 (2006.01)

According to International Patent Classification (IPC) or to both national classification and IPC

**B. FIELDS SEARCHED**

Minimum documentation searched (classification system followed by classification symbols)

C02F 1/00, 3/00, 3/30, G21F 9/00, 9/04, G21G 5/00, C12N 1/00, 5/00

Documentation searched other than minimum documentation to the extent that such documents are included in the fields searched

Electronic data base consulted during the international search (name of data base and, where practicable, search terms used)

NCBI, PubMed, PatSearch (RUPTO internal), USPTO, WIPO, PAJ, Esp@cenet, PCT online, USPTO DB, CIPO(Canada PO), SIPO DB, KIPRIS

**C. DOCUMENTS CONSIDERED TO BE RELEVANT**

| Category* | Citation of document, with indication, where appropriate, of the relevant passages | Relevant to claim No. |
| --- | --- | --- |
| Y | KHIZHNIAK T.V. Bakterialnaya transformatsiya i immobilizatsiya tiazhelykh metallov i radionuklidov. Avtoreferat dissertatsii na soiskanie uchenoi stepeni doktora biologicheskikh nauk, M., 2013, p.1-49, particularly p.5-7, 12-14, 18, 22, 24, 41 | 1-7 |
| Y | RU 2052223 C1 ( TOVARISCHESTVO S OGRANICHENNOI OTVETSTVENNOSTJU NAUCHNO-PROIZVODITELNOE OBEDINENIE «INTER-NART») 10.01.1996, the abstract, p. 3-7, the claims | 1-7 |
| Y | RU 2330339 C1 (GOSUDARTSVENNOE OBRAZOVATELNOE UCHREZHDENIE VYSSHEGO PROFESSIONALNOGO OBRAZOVANIYA «SANKT-PETERBURGSKY GOSUDARSTVENNY TEKHNOLOGICHESKY INSTITUT») 27.07.2008, p.4, line 12 | 1-7 |

☒ Further documents are listed in the continuation of Box C.　　☐ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 22 December 2014 (22.12.2014) | 29 January 2015 (29.01.2015) |

| Name and mailing address of the ISA/ RU　　　　　　　RU | Authorized officer |
| --- | --- |
| Facsimile No. | Telephone No. |

Form PCT/ISA/210 (second sheet) (April 2005)

y
b
14

## INTERNATIONAL SEARCH REPORT

| | International application No. |
|---|---|
| | PCT/RU 2014/000273 |

**C (Continuation).     DOCUMENTS CONSIDERED TO BE RELEVANT**

| Category* | Citation of document, with indication, where appropriate, of the relevant passages | Relevant to claim No. |
|---|---|---|
| Y | UA 10809 A (OBSCHESTVO S OGRANICHENNOI OTVESTVENNOSTJU NAUCHNO-PROIZVODSTVENNAYA FIRMA «BIOEK») 25.12.1996, the abstract, example, the claims | 1-7 |
| Y | RU 126327 U1 (OBSCHESTVO S OGRANICHENNOI OTVETSTVENNOSTJU «PLANTATSIIA» et al.) 27.03.2013, the abstract, the claims | 5 |
| A | RU 2090944 C1 (BELOIARSKAYA ATOMNAYA ELEKTROSTATSIYA et al.) 20.09.1997, p.6, lines 14-24 | 1-7 |

Form PCT/ISA/210 (continuation of second sheet) (April 2005)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- RU 2255906

- US 4721596 A

**Non-patent literature cited in the description**

- **VAN BRUNT J. ; CALDWELL J. H. ; HAROLD F. M.** Circulation of potassium across the plasma membrane of Blastocladiella emersonii: K-chanel. *J. Bacteriol.,* 1982, vol. 150 (3), 1449-1561
- **V.I.VYSOTSKII ; M.V.VYSOTSKYY.** Coherent correlated states and low-energy nuclear reactions in non stationary systems. *European Physical Journal. A,* 2013, vol. 49 (8), 99

- **V.I.VYSOTSKII ; S.V.ADAMENKO ; M.V.VYSOTSKYY.** Acceleration of low energy nuclear reactions by formation of correlated states of interacting particles in dynamical systems. *Annals of Nuclear Energy,* 2013, vol. 62, 618-625